# EUROPEAN PATENT APPLICATION

(11) **EP 0 864 298 A2**
(43) Date of publication of application: **16.09.1998**
(21) Application number: 98301898.7
(22) Date of filing: 13.03.1998
(51) Int. Cl.: A61C 5/00, A61N 5/04

(54) **Tooth improving apparatus and tooth improving material**

(30) Priority: 14.03.1997 JP 82343/97; 26.03.1997 JP 93222/97; 11.04.1997 JP 110241/97; 27.02.1998 JP 48312/98; 27.02.1998 JP 48381/98
(71) Applicant: Egawa Corporation, Nagoya-shi, Aichi-ken (JP)
(72) Inventor: Nonomura, Yuusuke, Nagoya-shi, Aichi-ken (JP)
(74) Representative: Paget, Hugh Charles Edward

(57) **Abstract**

A tooth-improving apparatus, including an electromagnetic-wave emitter (10, 20, 30, 40, 50) which emits, toward at least one of a tooth (S) and a tooth-improving material (6, 36) which is applied to a surface of the tooth, at least one electromagnetic wave having at least one resonance wavelength of at least one substance present in said at least one of the tooth and the tooth-improving material. A tooth-improving material (6, 36) for being applied to a surface of a tooth and subjected to at least one electromagnetic wave having at least one resonance wavelength of at least one substance present in at least one of the tooth and the material.

## Description

### BACKGROUND OF THE INVENTION

### Field of the Invention

The present invention relates to an apparatus and a material which are suitable for improving a tooth of a living subject such as a human being or an animal.

### Related Art Statement

A tooth includes enamel in its superficial portion or surface layer. The dental enamel is almost constituted by hydroxyapatite crystals, though it additionally contains organic substances in small amounts. The hydroxyapatite crystals form enamel rods which have a diameter of 3 to 5 µm and which continuously extend from the boundary between the enamel and the dentin to the outer surface of the tooth.

The outer surface of the enamel is exposed to plaque, saliva, food, drink, quick temperature change, etc. Therefore, in the range from the outer surface, to the depth of several tens of microns to one hundred microns, of the enamel, the structures of hydroxyapatite crystals are random or incomplete, that is, some crystals have lattice defects, some crystals have foreign matters such as CO₂, Mg, carbon, etc., or the degree of crystallization of some crystals is low.

Because those incomplete crystals present in the enamel of the tooth are weak chemically and physically, the tooth is easily subject to caries due to the acid produced by bacteria and additionally is easily subject to wearing.

By the way, there has been a strong demand that the outer surface of a tooth be coated with a material similar to the enamel of the tooth for improving or whitening the surface of the tooth. However, there has been no means for satisfying this demand.

### SUMMARY OF THE INVENTION

It is therefore an object of the present invention to provide a tooth-improving apparatus which improves a tooth by crystallizing at least one of the tooth itself and a tooth-improving material which is applied to a surface of the tooth.

It is another object of the present invention to provide a tooth-improving material which is applied to a surface of a tooth and which is subjected to an electromagnetic wave.

It is another object of the present invention to provide a use of an electromagnetic-wave emitter which emits an electromagnetic wave, for improving a tooth.

It is another object of the present invention to provide a use of a tooth-improving material for improving a tooth.

It is another object of the present invention to provide a tooth-coating method for coating a tooth.

The invention includes both medical and non-therapeutic (e.g. cosmetic) treatments.

According to a first aspect of the present invention, there is provided a tooth-improving apparatus, comprising an electromagnetic-wave emitter which emits, toward at least one of (a) a tooth and (b) a tooth-improving material which is applied to a surface of the tooth, at least one electromagnetic wave having at least one resonance wavelength of at least one substance present in said at least one of the tooth and the tooth-improving material. In the case where the electromagnetic wave ("E wave") emitted by the E-wave emitter resonates with at least one substance (e.g., hydroxyapatite crystals) present in the superficial portion of the tooth, i.e., in the surface layer of the enamel of the tooth, the energy of the E wave is absorbed by, e.g., the hydroxyapatite crystals. Accordingly, the hydroxyapatite crystals constituting the enamel are re-crystallized, and the surface layer of the enamel is improved. Thus, the surface of the enamel becomes more resistant to not only acid or caries but also wearing. More specifically described, some random or incomplete hydroxyapatite crystals present in the surface layer of the enamel are re-crystallized or completed, so that the surface layer of the enamel is strengthened. In addition, since the impurities such as carbon contained in the surface layer of the enamel are removed by the resonance of the crystals with the E wave, the strength of the surface layer of the tooth is increased. Regarding some hydroxyapatite crystals which are bonded with foreign substances, those foreign substances are removed by the resonance of the crystals with the E wave, and those crystals are re-crystallized with necessary substances present therearound. In the case where the E wave emitted by the E-wave emitter resonates with the tooth-improving material applied to the surface of the tooth, hydroxyapatite crystals and/or similar crystals are grown from the substance or substances contained in the tooth-improving material, or hydroxyapatite crystals contained in the tooth-improving material are re-crystallized. In the case where the E wave emitted by the E-wave emitter resonates with both the tooth and the tooth-improving material applied to the surface of the tooth, the hydroxyapatite crystals of the tooth are re-crystallized on one hand, and new hydroxyapatite crystals are grown from the substance or substances contained in the tooth-improving material, or the hydroxyapatite crystals contained in the material are re-crystallized on the other hand, so that the tooth is united with the tooth-improving material. A crack produced in the tooth can be filled with the tooth-improving material. Thus, a coating layer consisting of the hydroxyapatite crystals and/or the similar crystals is formed on the surface of the tooth. Since the hydroxyapatite crystals have a white color, the tooth coated with the coating layer has a white color. In the case where the tooth-improving material contains a color agent such as pigment, the tooth is colored with a desirable color.

According to a preferred feature of the first aspect of the invention, the electromagnetic-wave emitter comprises an electromagnetic-wave generator which generates an initial electromagnetic wave, and a resonance device which provides, from the initial electromagnetic wave, the electromagnetic wave having the resonance wavelength. In the case where the E wave having the resonance wavelength of the hydroxyapatite crystals is emitted toward the tooth, the re-crystallization or growing of hydroxyapatite crystals progresses in a short time.

According to another feature of the first aspect of the invention, the electromagnetic-wave generator comprises a globar which emits, as the initial electromagnetic wave, a global electromagnetic wave having a plurality of wavelengths including the resonance wavelength, and wherein the resonance device comprises a filter which transmits, from the global electromagnetic wave, only a wave having the resonance wavelength and does not transmit the other waves having the other wavelengths, thereby providing the electromagnetic wave having the resonance wavelength. In this case, since the globar as a light source and the filter are produced at low cost, the E-wave generator can be produced at low cost. The filter may be an interference filter.

According to another feature of the first aspect of the invention, the resonance device comprises an interference device which derives, from the initial electromagnetic wave, two waves having a phase difference therebetween and causes the two waves to interfere with each other, thereby providing the electromagnetic wave having the resonance wavelength. In this case, the E wave having the resonance wavelength can permeate to the interface or boundary between the surface of the tooth and the tooth-improving material applied to the surface of the tooth. Thus, hydroxyapatite crystals, for example, are easily crystallized at the boundary, and the tooth is coated with the coating layer in a short time.

According to another feature of the first aspect of the invention, the electromagnetic-wave emitter comprises a pulse-wave emitter which emits a plurality of pulse waves as the electromagnetic wave having the resonance wavelength, and wherein the electromagnetic-wave emitter further comprises interval-control means for changing an interval between the pulse waves emitted by the pulse-wave emitter. When the interval between the two pulse waves incident to the tooth is controlled or changed to an appropriate time, the phases of vibration of molecules being excited are matched with one other, so that an E wave having a great energy is radiated from the tooth. This great energy of the E wave causes the re-crystallization of, e.g., the hydroxyapatite crystals constituting the enamel, thereby improving the surface of the tooth. Since the great energy is produced in the tooth when the two pulse waves are emitted at an appropriate interval, each of the pulse waves emitted by the pulse-wave emitter may have a small energy. Thus, the load of the E wave exerted to the tooth is reduced.

According to another feature of the first aspect of the invention, the tooth-improving apparatus further comprises an image taking device which takes an image represented by an electromagnetic wave radiated from said at least one of the tooth and the tooth-improving material, and a monitor device which displays the image taken by the image taking device. In this case, an observer such as a dentist can obtain, from the image displayed on the monitor device, the information relating to the inside of the tooth and/or the gum therearound. That is, the observer can observe the internal focus of the tooth or the gum that cannot be observed by seeing the external surface of the tooth or the gum with his or her naked eyes. Thus, the dentist can make a correct diagnosis. In addition, since no contact element such as a probe is used, the tissue of the tooth or the gum is not damaged.

According to another feature of the first aspect of the invention, the resonance wavelength falls in a range from 8.8 to 10.4 µm. More preferably, the resonance wavelength falls in a range from 9.1 to 10.1 µm.

According to another feature of the first aspect of the invention, the resonance wavelength is a resonance wavelength, 9.6 µm, of a phosphoric group (i.e., phosphorus-oxygen bond thereof) of a hydroxyapatite crystal present in the at least one of the tooth and the tooth-improving material. However, the resonance wavelength may be a resonance wavelength of another substance (Ca²⁺, OH⁻) constituting the hydroxyapatite crystal.

According to a second aspect of the present invention, there is provided a tooth-improving material for being applied to a surface of a tooth and subjected to at least one electromagnetic wave having at least one resonance wavelength of at least one substance present in at least one of the tooth and the material. When the surface of the enamel or the dentin of the tooth is re-crystallized by the E wave having the resonance wavelength of at least one substance (e.g., hydroxyapatite crystals) present in the tooth, the substance or substances contained in the tooth-improving material applied to the surface or the tooth is or are used for re-crystallizing the enamel or the dentin. Thus, the tooth-improving material helps the re-crystallization of the enamel or the dentin. The at least one substance present in the at least one of the tooth and the material may be atoms or ions, molecules, crystals, or structures such as enamel or dentin.

According to a preferred feature of the second aspect of the invention, the tooth-improving material has a fluidity, and the at least one substance comprises at least one of calcium ion and phosphoric ion. The calcium ion and the phosphoric ion are essential constituents of the hydroxyapatite crystals present in the enamel and the dentin of a tooth.

According to another feature of the second aspect of the invention, the at least one substance comprises a hydroxyapatite crystal. The hydroxyapatite crystal may be given in the form of powder.

According to another feature of the second aspect of the invention, the at least one substance comprises fluorine. When the tooth is irradiated with the E wave having the resonance wavelength, the hydroxyl group contained in some hydroxyapatite crystals present in the tooth is substituted with the fluorine contained in the tooth-improving material. Thus, the strength of the surface of the tooth is improved.

According to a third aspect of the present invention, there is provided a use of an electromagnetic-wave emitter for emitting, toward at least one of (a) a tooth and (b) a tooth-improving material which is applied to a surface of the tooth, at least one electromagnetic wave having at least one resonance wavelength of at least one substance present in said at least one of the tooth and the material.

According to a fourth aspect of the present invention, there is provided a use of a tooth-improving material for being applied to a surface of a tooth and subjected to at least one electromagnetic wave having at least one resonance wavelength of at least one substance present in at least one of the tooth and the material.

According to a fifth aspect of the present invention, there is provided a tooth-coating method, comprising the steps of coating a tooth with a tooth-coating material, and emitting, toward the tooth-coating material, at least one electromagnetic wave having at least one resonance wavelength of at least one substance present in at least one of the tooth and the material.

### BRIEF DESCRIPTION OF THE PREFERRED EMBODIMENTS

The above and optional objects, features, and advantages of the present invention will be better understood by reading the following detailed description of the preferred embodiments of the invention when considered in conjunction with the accompanying drawings, in which:
Fig. 1A is a diagrammatic view of a tooth-improving device to which the present invention is applied;
Fig. 1B is a view of a Mach-Zehnder-type interference device which may be employed in the tooth-improving device of Fig. 1A;
Fig. 1C is a view of a Michelson-type interference device which may be employed in the tooth-improving device of Fig. 1A;
Fig. 2A is a diagrammatic view of another tooth-improving device as a second embodiment of the invention;
Fig. 2B is a graph representing two pulse waves, Al and A2, emitted at a time interval, t, by the tooth-improving device of Fig. 2A;
Fig. 2C is a graph representing an energy of an electromagnetic wave, B, radiated from a tooth, S, in response to the two pulse waves emitted by the tooth-improving device of Fig. 2A;
Fig. 3 is a view of a tooth-improving material applied to a surface of a tooth S;
Fig. 4A is a diagrammatic view of another tooth-improving device as a third embodiment of the present invention;
Fig. 4B is a view of a tooth-improving material applied to a surface of a tooth S;
Fig. 5A is a diagrammatic view of another tooth-improving device as a fourth embodiment of the present invention;
Fig. 5B is a view of a Mach-Zehnder-type interference device which may be employed in the tooth-improving device of Fig. 5A;
Fig. 5C is a view of a Michelson-type interference device which may be employed in the tooth-improving device of Fig. 5A;
Fig. 6A is a diagrammatic view of another tooth-improving device as a fifth embodiment of the present invention;
Fig. 6B is a graph representing two pulse waves A1, A2 emitted at a time interval t by the tooth-improving device of Fig. 6A; and
Fig. 6C is a graph representing an energy of an electromagnetic wave B radiated from a tooth S in response to the two pulse waves emitted by the tooth-improving device of Fig. 6A.

### DETAILED DESCRIPTION OF THE PREFERRED EMBODIMENTS

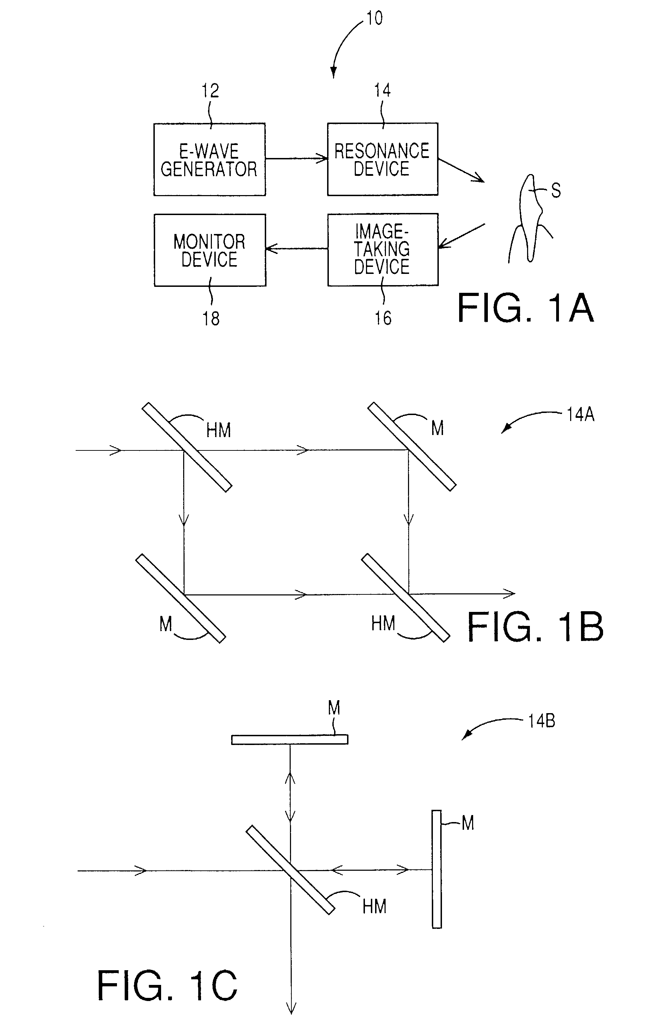

Referring first to Fig. 1 (Figs. 1A, 1B, and 1C), there will be described a first embodiment of the present invention which relates to a tooth improving apparatus 10.

The tooth improving apparatus 10 includes an electromagnetic-wave ("E-wave") generator 12 which generates an initial electromagnetic wave ("E wave"), and a resonance device 14 which provides, from the initial E wave, an E wave ("resonance wave") having a resonance wavelength of hydroxyapatite crystals which are present in a tooth, S, of a living subject such as a human being or an animal. The resonance wavelength falls in the range from 8.8 to 10.4 µm, and is preferably equal to 9.6 µm. The resonance device 14 emits the resonance wave toward an outer surface (i.e., superficial portion or surface layer) of the tooth S. In addition, the present apparatus 10 includes an image-taking device 16 which takes an image represented by an E wave radiated from the tooth S in response to the resonance wave, and a monitor device 18 which visualizes or displays the image taken by the image taking device 16.

The E-wave generator 12 may be provided by a laser-light emitter which emits, as the initial E wave, a coherent laser light (e.g., E wave) having a predetermined wavelength, which is equal to 633 nm in the present embodiment.

The resonance device 14 may be provided by a Mach-Zehnder-type E-wave interference device 14A as shown in Fig. 1B, or by a Michelson-type E-wave interference device 14B as shown in Fig. 1C. In Figs. 1B and 1C, symbol, M, designates mirrors each of which completely reflects an E wave input thereto, and symbol "HM" designates half mirrors each of which transmits a portion of an E wave input thereto and reflects the remaining portion of the same. Each of the interference devices 14A, 14B derives, from the laser light emitted by the laser-light emitter 12, two waves having a phase difference therebetween, and causes the two waves to interfere with each other, thereby providing the resonance wave. Each of the two interference devices 14A, 14B can be adjusted to a desirable resonance wavelength by changing one or more of the respective angles of the mirrors M and the half mirrors HM.

The wavelength of the E wave emitted by the resonance device 14 is equal to 9.6 µm (microns) that is the resonance wavelength of the hydroxyapatite crystals which constitute the enamel of the tooth S, or falls in the range of 9.6 ± 0.8. The resonance wave emitted from the resonance device 14 is directed to the outer surface of the tooth S, so that the resonance wave permeates into the superficial layer of the enamel of the tooth S, to the depth of more than several tens of microns.

The resonance device 14 includes an inlet through which the E wave generated by the E-wave generator 12 is input thereto, and an outlet through which the resonance wave is output therefrom. The inlet may be equipped with an angle adjusting device which is operable for adjusting the angle contained by the E wave input thereto and, similarly, the outlet may be equipped with an angle adjusting device which is operable for adjusting the angle contained by the resonance wave output therefrom.

The image-taking device 16 may be provided by an image sensor which detects an E wave radiated from the tooth S (i.e., wave reflected by, or transmitted through, the tooth S) in response to the resonance wave. For example, the image sensor may be an HgCdTe image sensor which is capable of detecting an infrared light and accordingly detecting an E wave radiated from the phosphoric groups of the apatites of the tooth S. However, other sorts of image sensors may be employed for taking an image represented by an E wave radiated from the tooth S.

The image-taking device 16 outputs an image signal representing the taken image represented by the E wave radiated from the tooth S, and the image signal is input to the monitor device 18, which displays, based on the image signal, the image of the tooth S corresponding to the image represented by the E wave radiated from the tooth S. In some cases, the monitor device 18 additionally displays images of the bone and/or the gum around the tooth S. In the case where the image represented by the E wave radiated from the tooth S is observed through the image-taking device 16 and the monitor device 18, better results can be obtained by using pulse waves than using a continuous wave as the resonance wave emitted by the resonance device 14.

Another image-taking device (not shown) may be employed for measuring the amount of energy of the E wave generated by the E-wave generator 12, or the amount of energy of the E wave radiated from the tooth S, and outputting a measurement signal representing the measured energy amount. In this case, a control device (not shown) is employed for controlling, based on the measurement signal supplied from the image-taking device, the E-wave generator 12 to generate an E wave having a controlled energy amount. To this end, the image-taking device may be provided by a single-picture-element HgCdTe image sensor which is capable of detecting an E wave corresponding to a single picture element only. Otherwise, the image-taking device may be provided by a low-price thermometer or temperature sensor.

Next, there will be described the operation of the tooth improving apparatus 10.

The E-wave generator 12 is operated to generate an E wave, and the resonance device 14 provides, from the E wave, a resonance wave which is emitted toward the outer surface of the tooth S, so that the resonance wave permeates into the depth of about several tens of microns in the superficial portion of the enamel of the tooth S.

Thus, the resonance wave resonates with the hydroxyapatites constituting the superficial portion of the enamel of the tooth S, so that the energy of the resonance wave is absorbed by the apatites. In this way, the apatites are re-crystallized and accordingly the physical and chemical properties of the superficial portion of the enamel is improved. More specifically described, in a natural state, the superficial portion of the enamel includes those hydroxyapatite crystals which are random or incomplete, i.e., have defects, and those random crystals are completed or corrected by the resonance with the resonance wave emitted from the resonance device 14.

That is, the superficial portion of the enamel of the tooth S is strengthened. The strengthening of the superficial portion of the enamel contributes to improving the acid-resistance of the tooth S, thereby preventing the tooth S from caries, and also contributes to reducing the wearing of the tooth S.

In the superficial portion of the enamel of the tooth S, there are some hydroxyapatite crystals which are bonded with, or contain, foreign matters such as CO₂, Mg, carbon, unnecessary phosphor, etc. Those foreign matters can be removed from those crystals which are subjected to the resonance wave. Additionally, those incomplete crystals are re-crystallized using necessary substances (e.g., calcium ions and phosphoric ions) present therearound, so that those crystals are completed. Thus, the superficial portion of the enamel is improved.

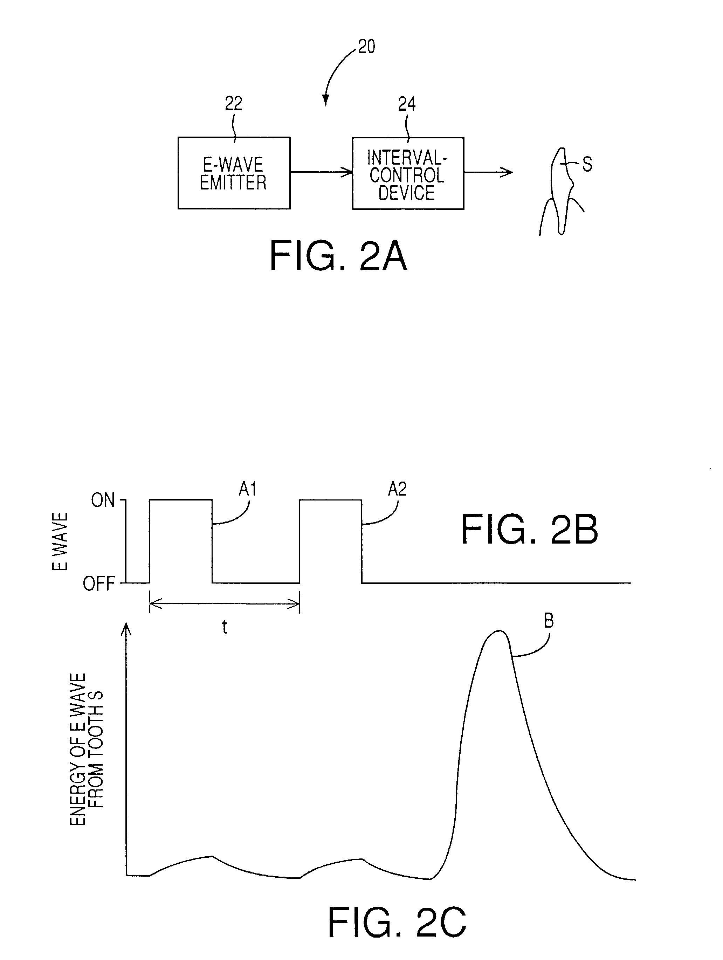

Referring next to Fig. 2 (Figs. 2A, 2B, and 2C), there will be described a second embodiment of the present invention which also relates to a tooth improving device 20.

The tooth improving device 20 includes an E-wave emitter 22 which emits an E wave, and an interval-control device 24 which controls or changes an interval at which the E-wave emitted by the E-wave emitter is directed to a tooth S.

The E-wave emitter 22 may be provided by a laser-light emitter which emits a coherent light having a predetermined wavelength which is equal to 9.6 µm (or falls in the range of 9.6 ± 0.8 µm) in the present embodiment. Since the wavelength of 9.6 ± 0.8 µm is the resonance wavelength of the hydroxyapatite crystals constituting the enamel of the tooth S, the E-wave emitter 22 functions as a resonance device which emits a resonance wave toward the tooth S. The E-wave emitter 22 includes an outlet which may be equipped with an angle-adjusting device which is operable for adjusting the angle contained by the resonance wave emitted therefrom.

The interval-control device 24 divides the resonance wave emitted by the E-wave emitter 22, into a first pulse wave, A1, and a second pulse wave, A2, as shown in Fig. 2B, and can automatically change a time of interval between the two pulse waves A1, A2, to a desirable time. The interval-control device 24 may include (a) a shutter member which can be opened ("ON") and closed ("OFF") to permit the resonance wave emitted from the E-wave emitter 22 to travel to the tooth S and shut off the same; (b) a drive device which opens and closes the shutter member by moving the same; and (c) a computer which changes the timing at which the drive device opens and closes the shutter member. Otherwise, the interval-control device 24 may be provided by a Q switch which regularly increases and decreases the energy of the resonance wave emitted by the E-wave emitter 22. Moreover, the control device 24 and the E-wave emitter 22 may be provided by a pulse laser which emits pulse laser lights at a controlled interval.

The interval-control device 24 can automatically determine a desirable interval in such a manner that when the interval between the two pulses A1, A2 gradually increases from zero, the control device 24 determines, as the desirable interval, an interval, t, at which the energy of an E wave, B, radiated from the tooth S in response to the two pulse waves A1, A2 becomes maximum, as shown in Fig. 2C. This interval t depends on an individual tooth or person. The control device 24 includes a HgCdTe image sensor or a thermometer, described above, which measures the energy of the E wave B.

The control device 24 may be adapted to successively emit two or more pairs of pulse waves A1, A2, at the determined interval, toward the tooth S. In this case, the control device 24 may be adapted to be able to change the number of successive emissions of the pairs of pulse waves A1, A2, to a desirable value.

Like the tooth-improving device 10 shown in Fig. 1, the tooth-improving device 20 may be equipped with an image-taking device 16 which takes an image represented by an E wave radiated from the tooth S in response to the resonance wave, and a monitor device 18 which displays the image taken by the image taking device 16, so that a dentist or an operator can observe the image of the tooth S displayed on the monitor 18.

When the first and second pulse waves A1, A2 are applied at an appropriate interval to the tooth S, the E wave B having the great energy is radiated from the tooth S. This great energy of the E wave B causes the re-crystallization of the hydroxyapatite crystals constituting the enamel of the tooth S, thereby improving the enamel of the tooth S.

The great energy of the E wave B is obtained from the first and second pulses A1, A2 each of which has a small energy. Since the resonance wave emitted by the E-wave emitter 22 has only a small energy, the tooth S is prevented from being excessively heated by the resonance wave, so that the dental pulp or other tissues around the tooth S are not damaged.

In each of the first and second embodiments, the tooth-improving apparatus 10, 20 improves the surface layer of the enamel of the tooth S. However, the apparatus 10, 20 may be used for emitting the resonance wave toward an exposed surface layer of the dentin of the tooth S (e.g., hypersensitive dentin) and thereby strengthening the exposed dentin.

The first and second embodiments may be combined with each other. That is, the E-wave generator 12 and the resonance device 14 employed in the first embodiment may be employed as the E-wave emitter 22 in the second embodiment. In this case, since a great energy is produced in the tooth S, the E-wave generator 12 may generate an E wave having a small energy.

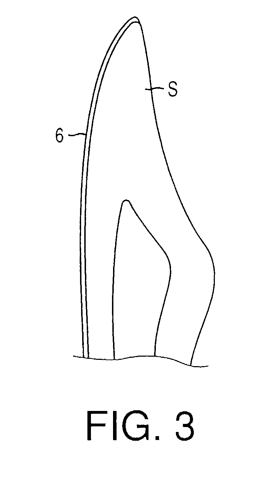

As shown in Fig. 3, a tooth-improving material 6 including one or more substances constituting the tooth S may be applied to the surface of the tooth S, and the tooth-improving apparatus 10, 20 may emit the E wave toward the tooth S and the tooth-improving material 6 applied to the tooth S. This material 6 may consists of aqueous solution, or powder, including, as the substance or substances constituting the tooth S, at least one of Ca²⁺, PO₄ ³⁻, and hydroxyapatite crystals.

In the above case, when the surface layer of the tooth S is re-crystallized by the resonance wave emitted by the apparatus 10, 20, the substance or substances contained in the material 6 applied to the surface of the tooth S is or are used for the re-crystallization of the enamel. That is, the tooth-improving material 6 includes the substance or substances needed for re-crystallizing the enamel, and accordingly the material 6 can help the re-crystallization of the enamel.

The tooth-improving material 6 may include fluorine and/or one or more other tooth-strengthening agents. In this case, when the tooth S is irradiated with the resonance wave, the hydroxyl group of the hydroxyapatite present in the tooth S is substituted by the fluorine. Thus, the surface of the tooth S is strengthened. The material 6 may include both one or more substances (Ca²⁺, PO₄ ³⁻, and/or hydroxyapatite) constituting the tooth S, and one or more tooth-strengthening agents such as fluorine.

In each of the first and second embodiments, the laser-light emitter 12, 22 is employed for emitting the resonance wave toward the tooth S. However, other sorts of E-wave emitters may be employed so long as they can provide an E wave having a resonance wavelength of the hydroxyapatite crystals present in the tooth S. The E wave may be laser light, natural light, radio wave, x ray, medium wave, ultraviolet ray, infrared ray, or visible ray, and may be coherent wave or incoherent wave. The E wave may take any shape, such as pulse wave, continuous wave, burst wave, sine wave, triangular wave, saw-tooth wave, damped-oscillation wave, or sin/x wave, so long as it can meet the aim of the invention.

The initial wave to be interfered with may be any E wave having any wavelength so long as it can provide the target wavelength. The target wavelength may be changed, as needed, depending on the nature of a living body such as a patient. For example, if a wavelength in the order of 200 nm is used for a tooth, a diagnosis can be made on the tooth at the level of electrons; and since skin, gum, and various soft tissues have an absorption band such as about 6 µm or 2.7 µm, a diagnosis can be made at the level of molecules. Moreover, in a specific magnetic field, a diagnosis can be made at the level of atoms. In the case where the interference wave is a radio wave, an antenna, a waveguide tube, or an electromagnetic-field lens may be used. An infrared light may be received by an antenna. The light source may be any sort of light source such as a globar, a lamp, or an LED (light emitting diode), so long as it can be applicable to the invention.

In each of the two embodiments, an emission-control device may be employed for controlling the E-wave generator 12 or the E-wave emitter 22 to generate or emit an E wave having an amplitude modulation, a frequency modulation, or a phase modulation. The frequency or wavelength may be continuously or stepwise changed by ± δ.

The operator can freely locate the parts of the interference device 14A or 14B. The interference wave, or the E wave from the E-wave generator 12, may be guided by a wave-guiding element such as optical fiber and be directed to the tooth S. The interference device 14A or 14B may be so located that the interference occurs in the vicinity of the tooth S. Since an optical fiber transmitting a visible light can be produced at low cost, it is preferable to use the visible light till it is interfered with.

The tooth-improving apparatus 10, 20 may be used by being attached to a nightguard or a biteplate when a living subject sleeps. In this case, it is preferable to use an optical fiber and/or a battery.

Two or more pulse E waves may be applied at an appropriate interval to the tooth S, or a continuous wave may be applied to the tooth S. The first and second pulse waves A1, A2 may be used for making an observation by using E waves which are reflected from, or transmitted through, the tooth S. The interval between the first and second pulse waves A1, A2 may be changed for adjusting the phase matching. A single pulse wave may be used for observing the re-radiation thereof, or the re-radiation of one pulse wave may be compared with that of another pulse wave.

The E wave directed to the tooth S may have a single wavelength or a plurality of wavelengths. The E-wave interference may be (a) an interference of three or more E waves from three or more directions, or (b) a multiple interference obtained by superimposing a plurality of interferences on each other, or a combination of the two sorts of interferences (a) and (b). In these cases, respective sites of a complex tissue can be finely irradiated independent of each other. A wavelength before being changed may be used for excitation. Those waves may be subjected to Fourier synthesis.

In the case where an interference wave is applied to the tooth S, any interference wavelength, any interference method, or any interference device may be employed so long as an interference is given to the tooth S. In this case, the light-polarization conditions, the frequency modulation, or the tooth-irradiation position can be easily controlled. Thus, the interference wave can be easily controlled to permeate into deeper portions of the tooth. In addition, the tooth S can be irradiated from its front surface, from its rear side, or from considerably oblique positions. Thus, the excitation wavelength can be selected in a wide range, and the excitation site can be easily reached.

In the first embodiment, the wavelength of the interference wave is controlled or changed by changing the angle of interference. However, the wavelength of the interference wave may be controlled by changing the frequency or wavelength of any one of the waves (oscillation waves) to be interfered with, or by individually or simultaneously changing the respective frequencies or wavelengths of all those waves. Otherwise, the interference wave may be controlled by using a diffraction grating or a double-refractive element. An optimum excitation wave may be determined based on the response of the tooth S, or based on the prescribed conditions of devices.

A light (excitation light) reflected by a phase conjugate mirror may be used as an E wave to irradiate the tooth S. Conversely, a light radiated from the tooth S may be reflected by a phase conjugate mirror, and the light reflected may be received by a light receiving or detecting device. Alternatively, a heterodyne detection may be employed. In addition to these techniques, a wavelength transformation using a nonlinear crystal may be used with an electromagnetic-field generating means. In the case where these wavelength transforming means are used solely or in combination, the distortion of the E wave by the propagation medium present in the propagation path (e.g., the substances present around the site where the radiation wave to be observed is produced, or the refractive substances present in the air) is corrected, and the change or transformation of the wavelength advantageously occurs in the vicinity of the tooth S, at the surface of the same S, or inside the same S. In the wavelength transformation employed in the illustrated embodiments, the transformed wavelength is longer than the initial wavelength. Accordingly, the initial wave has a lower energy than that of the transformed wave. However, in these transformation manners, the wavelength may be shortened. Therefore, the transformed wave may have a higher energy.

If the waves to be interfered with have wavelengths which easily transmit through the tooth S, they are very effective to an undermining focus, or a focus having an undercut. In many cases, caries does not start at the surface of the tooth S, but starts under the surface. In those cases, those waves are suitable for carrying out annealing under the surface, or making a diagnosis on that focus. A polarized light such as a linearly polarized light, an elliptically polarized light, or circularly polarized light may be controlled to improve the depth over which the light can permeate into the tooth S. In the case where saliva and/or a water or the like emitted from a cutting device are/is present in the mouth of the patient, the linearly polarized light is advantageous. A wave plate may be placed, as needed, for controlling the optical path, or controlling the polarized light. In addition, a shutter member may be inserted in the optical path for controlling the pattern or manner in which the tooth S is irradiated.

Excitation is likely to be understood as involving an increase in energy level. However, as far as the present invention is concerned, the excitation is defined as including a negative excitation involving a decrease in energy level. For example, if an external wave which can interfere with one or more internal waves present in the substances constituting the tooth S is applied to the tooth S to interfere with the internal wave or waves, a potential energy inside the tooth may be decreased, or one or more functions of the tooth S may be controlled. In addition, a more efficient excitation can be done, and a predetermined portion can be surely excited. One or more of these wavelength transforming means may be employed.

The E wave may be used for the polymerization of, e.g., a photo-polymerizable resin, a thermo-polymerizable resin, or a chemo-polymerizable resin. In these cases, a high degree of polymerization can be obtained, and a better polymerization can be obtained at a deep portion. Generally, a photo-polymerizable resin which is used in the mouth is polymerized with a wavelength which tends to injure the eyes. However, the present invention can solve this problem, by using a wave having a wavelength which does not injure the eyes, till the wavelength is transformed.

In the illustrated embodiments, the image-taking device 16 is provided by the HgCdTe image-taking elements. However, other sorts of image-taking elements such as CCD (charge-coupled device) camera, InAs, PbSnTe, PbS, CdS, CdSe, PZT, LiTaO₃ elements, a thermopile, or a bolometer may be employed. Alternatively, an antenna may be employed to obtain the same effect.

An E wave to be observed may be a wave which is reflected by, or transmitted through, the tooth S.

The waveform detected by the signal receiving means 16 may be subjected to frequency analysis, such as Fourier analysis, wavelet analysis, or correlation analysis, to improve accuracy with respect to a specific wavelength. The signal receiving means, or the subsequent electric circuit or analyzing computer, may be equipped with a filter. However, the signal receiving means may be omitted, or otherwise may be equipped with a wavelength selecting means.

The display device 18 may display a monochromatic or full-color image which may, or may not, have a gradation. Moreover, the display 18 may display the image which is represented by the E wave radiated from the tooth S and which is synthesized with a natural image of the tooth S. In the latter case, the synthesized image is suitable for diagnosis or monitoring of the tooth S. The display 18 may be connected to another diagnosing device.

As the re-crystallization of the hydroxyapatite progresses with the resonance wave, the resonance wavelength of the hydroxyapatite crystals changes or shifts. Therefore, in the first embodiment shown in Fig. 1, it is preferred to employ a control device which measures the intensity of the E wave radiated from the tooth S and controls, based on the measured intensity, the resonance device 14 or the interference device 14A, 14B to change the wavelength of the E wave emitted thereby to match the shifting of the resonance wavelength of the hydroxyapatite crystals.

In the second embodiment shown in Fig. 2, the E-wave emitter 22 and the interval-control device 24 cooperate with each other to emit two pulse waves toward the tooth S. However, those devices 22, 24 may be adapted to emit three or more pulse waves toward the tooth S.

The intensity may be associated with an index or a threshold. The frequency at which, e.g., the peak of phosphoric group shifts may be associated with a threshold, or the Q value of the resonance may be associated with a threshold. Only one or more of those thresholds may be selected and employed.

The shape of the peak may be continuously monitored by a signal-receiving means, and the Q value may be feedback controlled not to exceed a threshold to prevent the destruction of the crystals. This control may be either the setting of a destruction line or value, or the feedback control to the maximum Q value.

In the case where the hydroxyapatite crystals are re-crystallized using the pulse waves A1, A2, it can be thought that the molecular vibration excited by the first pulse A1 is phase matched (or phase inverted) by the second pulse A2 and therefore that a more coherent and efficient re-crystallization results. Thus, the re-crystallization of the crystals of the tooth S which has been impossible becomes possible, and the physical, chemical, and biological properties of the tooth S, such as the resistance of the tooth S to caries, wearing, and acid, are improved. Re-crystallization may be caused by exciting atom, electron, tissue structure, or crystalline structure at the level of tissue structure. A third pulse wave may be applied to the tooth S when an E wave is radiated by the phase matching. In the last case, the efficiency is improved by the near-saturation of energy.

In the above embodiments, the resonance peak of the phosphoric group is employed as the index of resonance wavelength. However, the operator can freely select any resonance peak or any excitation peak to emit, or freely select the maximum value, or the half value, of the peak as a reference emission wavelength. The present invention is applicable to other tissues or structures than teeth, so long as they contain crystals. The present invention is also applicable to other structures than crystals so long as they can be changed like re-crystallization.

The several sorts of wavelength-transforming means described above may be employed in combination.

As far as the present invention is concerned, the term "interference" covers beat which is caused in the case where interference angle is equal to 0 or 180 degrees. The resonance wave may be incident to the tooth S at any angle.

In the illustrated embodiments, if the tooth-improving apparatus 10, 20 outputs the resonance wave having a high power or energy, the apparatus 10, 20 functions as a tooth-cutting machine. In this case, since the apparatus 10, 20 can cut the tooth S with a smaller energy than a conventional machine, the tissues around the tooth S are less influenced and the operator is prevented from safe hazards. The tooth-cutting effect can be enhanced by using a tooth-destroying agent such as an acid.

The fine destruction and the re-crystallization may be alternately repeated for improving the strength of the tooth S. An agent for neutralizing the acid may be used.

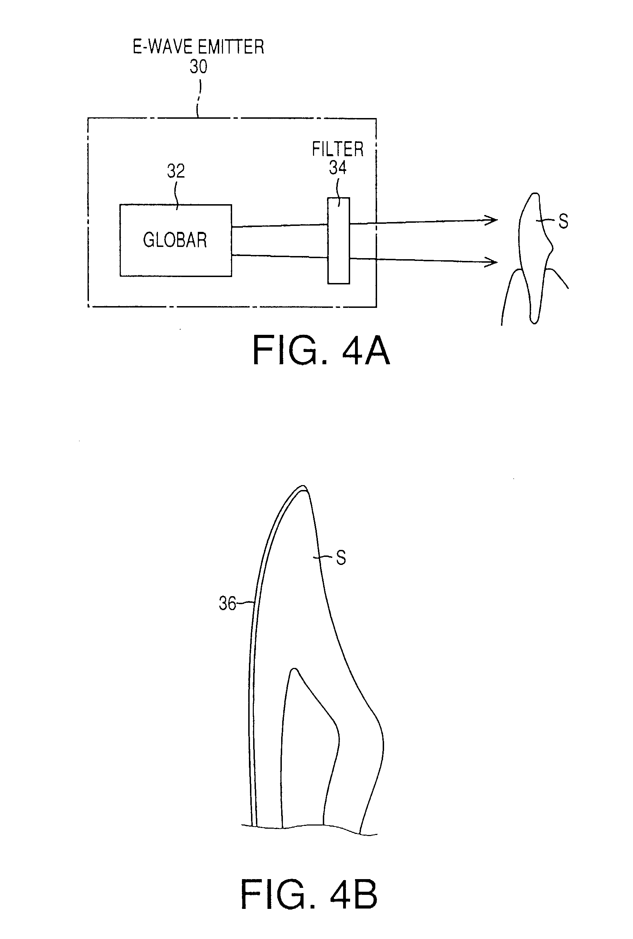

Referring next to Fig. 4 (Figs. 4A and 4B), there will be described a third embodiment of the present invention, which relates to a tooth-improving (or tooth-coating) apparatus and a tooth-improving (or tooth-coating) material 36 applied to a tooth S.

The tooth-improving apparatus includes an E-wave emitter 30 which emits an E wave and which includes a globar 32 as a light source, and a filter 34. The globar 32 emits a global electromagnetic wave including far infrared rays and infrared rays (including the resonance wavelength of hydroxyapatite crystals). The filter which transmits, from the global electromagnetic wave, only a wave having the resonance wavelength of the hydroxyapatite (hereinafter, referred to as the "resonance wave") and does not transmit the other waves having the other wavelengths. The resonance wavelength of the hydroxyapatite falls in the range of 9.6 ± 0.8 µm and is preferably equal to 9.6 µm. Thus, the E-wave emitter 30 emits only the resonance wave toward the tooth S coated with the coating material 36.

The amount or intensity of E wave emitted by the E-wave emitter 30 may be feedback controlled by a control device (not shown) which measures the intensity of an E wave radiated from the tooth S and changes, based on the measured intensity, the intensity of the E wave emitted by the emitter 30 to a desirable value. The control device may be equipped with a temperature sensor such as an HgCdTe sensor.

An image-taking device (not shown) such as an HgCdTe sensor may be employed for taking an image represented by the E wave radiated from the tooth S, and supplying an image signal representing the taken image to a monitor device (not shown), so that the monitor displays the image represented by the image signal received.

The coating material 36 includes one or more substances constituting the tooth S. More specifically described, in the present embodiment, the coating material 36 is a material which produces or grows, when being radiated with the resonance wave, hydroxyapatite crystals. The coating material 36 has such a fluidity that assures that the coating material 36 is thinly and uniformly applied to the surface of the tooth S. The coating material 36 consists of an aqueous solution containing oversaturated Ca²⁺ and PO₄ ³⁻ and a powder of hydroxyapatite crystals. The coating material 36 additionally includes (a) a small amount of coloring agent (e.g., pigment) for giving a color matching the color of the tooth S, or giving a desirable color of the user, and thereby preventing the tooth S from becoming too white; and (b) an adjusting agent for giving a degree of transparency to the tooth S.

The Ca²⁺, the PO₄ ³⁻, the powder of hydroxyapatite crystals, and the coloring agent may be stored in respective separate containers such as flexible tubes, and may be mixed with each other when being used. Otherwise, those substances may be applied in sequence to the surface of the tooth S.

When the coating material 36 applied to the surface of the tooth S is irradiated with the resonance wave by the E-wave emitter 30, the energy of the wave causes the ions Ca²⁺, PO₄ ³⁻ contained in the applied material 36 to grow apatite crystals (or similar crystals) at the boundary between the material 36 and the apatite of the tooth S. Thus, on the surface of the tooth S, the ions Ca²⁺, PO₄ ³⁻ of the material 36 are united with the hydroxyapatite of the tooth S itself. In addition, a portion of the apatite-crystal powder of the material 36 is united with the ions Ca²⁺, PO₄ ³⁻ of the material on one hand, and another portion of the apatite-crystal powder is united with the apatite of the surface of the tooth S.

Next, there will be described the operation of the tooth-coating apparatus described above.

First, the coating material 36 is thinly and uniformly applied to the surface of the tooth S. Then, the E-wave emitter 30 is operated to emit the resonance wave toward the tooth S coated with the coating material 36.

As a result, the coating material 36 is crystallized, and the hydroxyapatite (or similar substance) present in the surface of the tooth S is also crystallized. Thus, the boundary between the natural tooth S and the coating material 36 becomes extinct, that is, the tooth S and the material 36 are united with each other. In this way, the tooth S is coated with the coating material 36.

The tooth S may be coated two or more times, that is, coated with two or more coating layers each of which is formed of the coating material 36. In this case, after each coating layer is formed and before another coating layer is formed, the surface of each coating layer may be polished or smoothed.

Since the coating material 36 is used for forming the coating layer including the hydroxyapatite crystals and/or the similar crystals on the surface of the tooth S, the tooth S can be colored in white.

In addition, the surface of the tooth S is improved. That is, the resistance of the tooth S to acid is improved, and accordingly the tooth S is prevented from caries. The wearing of the tooth S is reduced. A crack or cavity produced in the tooth S can be filled with the hydroxyapatite crystals produced from the coating material 36. In the last case, too, the tooth S is made resistant to caries.

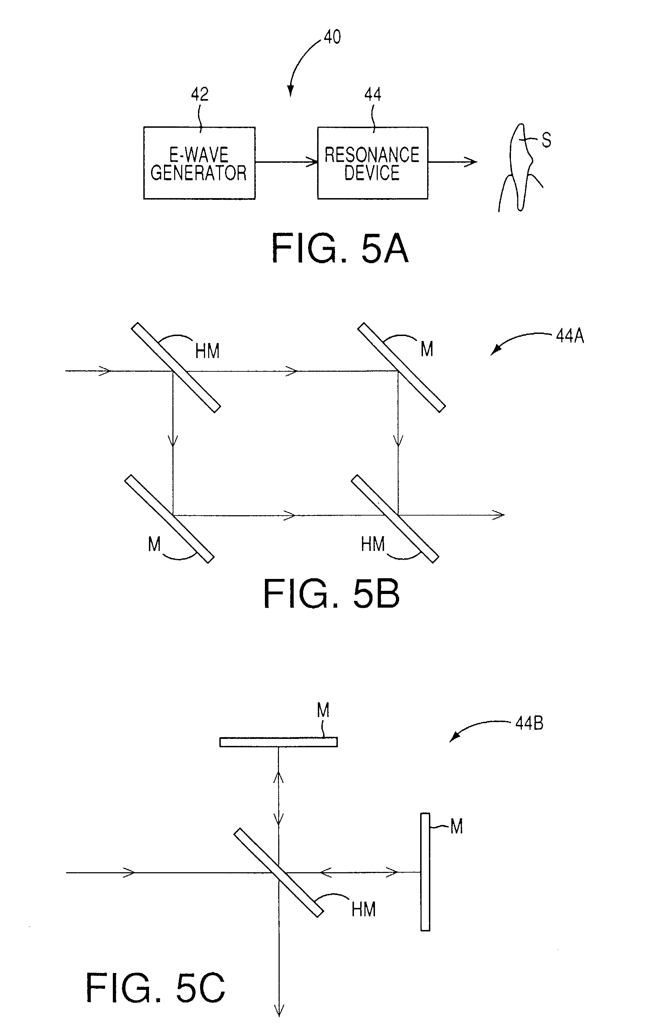

Referring next to Fig. 5 (Figs. 5A, 5B, and 5C), there will be described a fourth embodiment of the present invention which relates to a tooth-improving apparatus 40. The tooth-improving apparatus 40 includes an initial E-wave generator 42 which emits an E wave, and an interference device 44 which provides, from the initial E wave generated by the E-wave generator 42, an interference wave having a resonance wavelength of hydroxyapatite crystals present in a tooth S. The E-wave generator 42 may be provided by a laser-light emitter which emits, as the initial E wave, a laser light having a predetermined wavelength (e.g., 633 nm). The interference device 44 derives, from the initial E wave, two waves having a phase difference therebetween and causes the two waves to interfere with each other, thereby providing the resonance wave having the resonance wavelength. The resonance device 44 may be provided by a Mach-Zehnder-type E-wave interference device 44A as shown in Fig. 5B, or by a Michelson-type E-wave interference device 44B as shown in Fig. 5C. In Figs. 5B and 5C, symbol, M, designates mirrors each of which completely reflects an E wave input thereto, and symbol "HM" designates half mirrors each of which transmits a portion of an E wave input thereto and reflects the remaining portion of the same. Each of the two interference devices 44A, 44B can be adjusted to a desirable resonance wavelength by changing one or more of the respective angles of the mirrors M and the half mirrors HM.

The interference wavelength of the E wave emitted by the interference device 44 is equal to 9.6 µm that is the resonance wavelength of the hydroxyapatite crystals which constitute the enamel of the tooth S, or falls in the range of 9.6 ± 0.8 µm. The resonance wave emitted from the interference device 44 is directed to the outer surface of the tooth S, so that the resonance wave permeates into the coating material 36 and the surface layer of the tooth S.

The resonance device 44 includes an inlet through which the E wave generated by the E-wave generator 42 is input thereto, and an outlet through which the resonance wave is output therefrom. The inlet may be equipped with an angle adjusting device which is operable for adjusting the angle contained by the E wave input thereto and, similarly, the outlet may be equipped with an angle adjusting device which is operable for adjusting the angle contained by the resonance wave output therefrom.

When the tooth S is irradiated with the resonance wave emitted by the tooth-improving or tooth-coating apparatus 40, the resonance wave permeates into the coating material 36 and the surface layer of the tooth S. Thus, the hydroxyapatite and/or similar substance at the boundary between the surface of the tooth S and the coating material 36 is easily crystallized. Accordingly, the coating layer can be reliably formed on the surface of the tooth S in a short time.

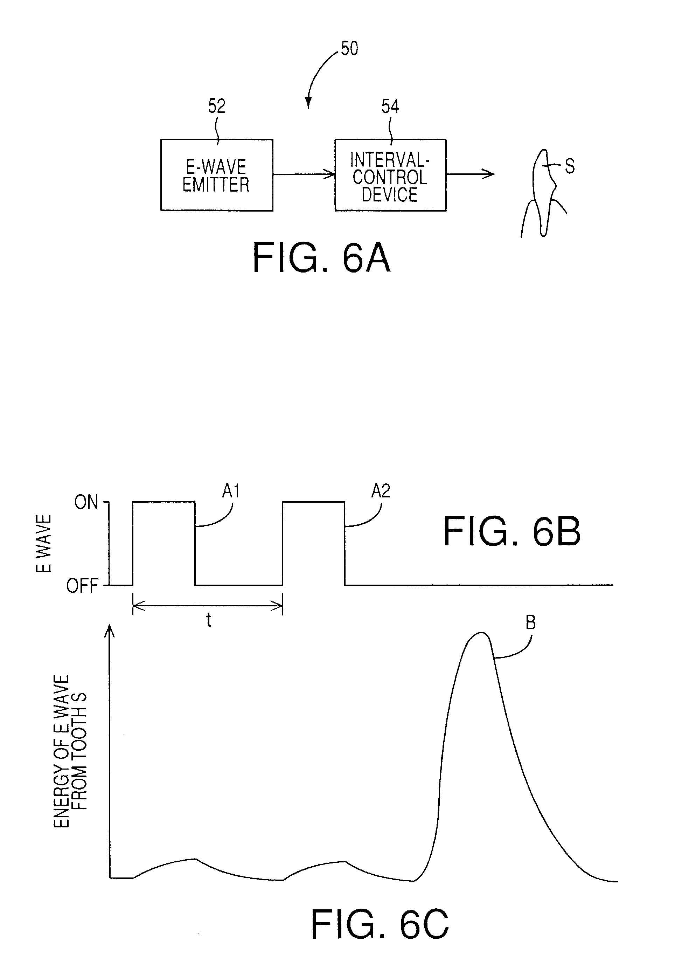

Referring next to Fig. 6 (Figs. 6A, 6B, and 6C), there will be described a fifth embodiment of the present invention which also relates to a tooth-improving (or tooth-coating) apparatus 50.

The tooth-coating apparatus 50 includes an E-wave emitter 52 which emits an E wave, and an interval-control device 54 which controls or changes an interval at which the E-wave emitted by the E-wave emitter is directed to a tooth S.

The E-wave emitter 52 may be provided by a globar 32 and a filter 34 employed in the third embodiment, or a laser emitter 42 employed in the fourth embodiment. The E-wave emitter 52 emits an E wave having a predetermined wavelength which is equal to 9.6 µm (or falls in the range of 9.6 ± 0.8 µm) in the present embodiment. The wavelength of 9.6 µm (or in the range of 9.6 ± 0.8 µm) is the resonance wavelength of the hydroxyapatite crystals constituting the enamel of the tooth S. Thus, the E-wave emitter 52 functions as a resonance device which emits a resonance wave toward the tooth S. The E-wave emitter 52 includes an outlet which may be equipped with an angle-adjusting device which is operable for adjusting the angle contained by the resonance wave emitted therefrom.

The interval-control device 54 divides the resonance wave emitted by the E-wave emitter 52, into a first pulse wave, A1, and a second pulse wave, A2, as shown in Fig. 6B, which are directed to the tooth S coated with the coating material 36 (not shown in Fig. 6A), and the device 54 can automatically change a time of interval between the two pulse waves A1, A2, to a desirable time. The interval-control device 54 may include (a) a shutter member which can be opened ("ON") and closed ("OFF") to permit the resonance wave emitted from the E-wave emitter 52 to travel to the tooth S and shut off the wave; (b) a drive device which opens and closes the shutter member by moving the same; and (c) a computer which changes the timing at which the drive device opens and closes the shutter member. Otherwise, the interval-control device 54 may be provided by a Q switch which increases and decreases the energy of the resonance wave emitted by the E-wave emitter 52 (globar 32 or laser emitter 42). Moreover, the control device 54 and the E-wave emitter 52 may be provided by a pulse laser device which emits pulse laser lights at a controlled interval.

The interval-control device 54 can automatically determine a desirable interval between the two pulse waves A1, A2 in such a manner that when the interval between the two pulses Al, A2 gradually increases from zero, the control device 54 determines, as the desirable interval, an interval, t, at which the energy of an E wave, B, radiated from the tooth S in response to the two pulse waves A1, A2 becomes maximum, as shown in Fig. 6C. The control device 54 includes a HgCdTe image sensor or a thermometer, described above, which measures the energy of the E wave B.

The control device 54 may be adapted to successively emit two or more pairs of pulse waves A1, A2, at the determined interval, toward the tooth S. In this case, the control device 54 may be adapted to be able to change the number of successive emissions of the pairs of pulse waves A1, A2, to a desirable value.

When the first and second pulse waves A1, A2 are applied at an appropriate interval to the tooth S, the E wave B having the great energy is radiated from the tooth S. This great energy of the E wave B causes the crystallization of the coating material 36 and the crystallization of the hydroxyapatite (or similar substance) at the boundary between the coating material 36 and the surface the tooth S, so that the crystallized coating material 36 is united with the hydroxyapatite of the tooth S. Thus, the tooth S is coated with the coating material 36.

The great energy of the E wave B is obtained from the first and second pulses A1, A2 each of which has a small energy. Since the resonance wave emitted by the E-wave emitter 52 has only a small energy, the tooth S is prevented from being excessively heated by the resonance wave, so that the dental pulp or other tissues around the tooth S are not damaged.

In each of the third to fifth embodiments, the tooth-coating apparatus 30, 40, 50 improves the surface layer of the enamel of the tooth S. However, the apparatus 30, 40, 50 may be used for emitting the resonance wave toward an exposed surface layer of the dentin of the tooth S (e.g., hypersensitive dentin) and thereby strengthening the exposed dentin. In this case, the dentinal tubules are closed and the surface of the dentin is enamelized. Therefore, the present apparatus 30, 40, 50 is more effective than known hypersensitive-dentin medicines. Two or more coating layers may be formed on the tooth S, for coating the exposed dentin of the tooth S.

The fourth and fifth embodiments may be combined with each other. That is, the E-wave generator 42 and the interference device 44 employed in the fourth embodiment may be employed as the E-wave emitter 52 in the fifth embodiment. In this case, since a great energy is produced in the tooth S, the E-wave generator 42 may generate an E wave having a small energy.

The tooth-coating material 36 may include fluorine and/or one or more other tooth-strengthening agents. In this case, when the tooth S is irradiated with the resonance wave, the hydroxyl group of the hydroxyapatite present in the tooth S is substituted by the fluorine. Thus, the surface of the tooth S is strengthened.

The tooth-coating material 36 may include, as one or more additive agents, one or more substances selected from the group including CaO, CaCO₃, Ca₃(PO₄)₂, CaHPO₄•2H₂O, CaCl₂, Ca(OH)₂, Ca(NO₃)₂, H₃PO₄, KH₂P0₄, (NH₄)₂HPO₄, NH₄OH, H₃PO₄, and NH₄H₂PO₄.

The coating material 36 may include a pH adjusting agent which may be, e.g., HNO₃ for acidification and may be, e.g. NH₄OH for basification.

The coating material 36 may include a catalyst which accelerates the crystallization thereof. The catalyst may be, e.g., gold, silver, platinum, palladium, or titanium, and may be used with another catalyst which utilizes a charging means or a polarizing technique. In the case where the catalyst is used in the coating material 36, it is preferable to precipitate the catalyst on the surface of the material 36 after the material 36 is irradiated with the E wave. To this end, tin ion, silane coupler, sol, or surfactant may be contained in the material 26.

The coating material 36 may have a low or high viscosity depending upon the substances contained therein and/or its applications. Each substance added to the material 36 may, or may not, be oversaturated (each substance may be present at a low concentration). The coating material 36 may take the form of powder. After a liquid coating material 36 is applied to, and crystallized on, the surface of the tooth S, another coating material having a high viscosity may be sintered to form a coating layer. Two or more coating layers may be formed on the tooth S.

An E-wave absorbing agent may be applied to the tissues around the tooth S. In this case, the coating layer is formed on the tooth S only, and is not formed on the tissues around the tooth S, even if the coating material 36 may be erroneously adhered to those tissues.

The coating material 36 may be used for filling a lacuna or a fissure of the tooth S. In this case, after the coating material filling the lacuna or fissure is irradiated with the E wave, a resin or a cement may, or may not, be used to cover the surface of the coating material. The coating apparatus 30, 40, 50 may emit the E wave two or more times. This preventive filling of the lacuna or fissure is safer than a conventional resin sealant, and is much more resistant to caries or wearing. The coating material 36 may be used for restoring a cavity or an abutment tooth or teeth, or lining, basing, or capping the pulp.

A resonance wavelength of a portion of the tooth S which is to be irradiated with the E wave emitted by the tooth-improving apparatus 10, 20, 30, 40, 50 may be measured in advance. In this case, the apparatus 10, 20, 30, 40, 50 may be adjusted to emit an E wave having the thus measured resonance wavelength. In addition, the degree of decalcification of that portion may be determined based on the measured resonance wavelength. A dentist can decide, based on the thus determined degree, whether it is necessary to treat that portion of the tooth S by coating it with the coating material 36, and/or determine a time duration during which the coating material 36 is irradiated with the E wave.

The present invention may be used for cutting or etching the tooth S using an acid such as lactic acid, phosphoric acid, maleic acid. The agent which operates when the tooth S is cut may be atoms such as H⁺ ions, or molecules which release H⁺ ions. Some substances may be cut using a base. In these cases, since a low-concentration solution can be used, the cutting or etching can be performed safely and efficiently. The coating layer once formed on the tooth S may be fused, and another coating layer may be formed on the fused layer. In this way, the quality of the tooth S is improved. The tooth S may be cleaned and/or etched before being coated with the coating material 36.

The foregoing description about the first and second embodiments and their variants are also true with the third to fifth embodiments.

The first to fifth embodiments and their variants may be implemented solely or in combination, and may be used in combination with one or more appropriate agents for enhancing one or more effects.

In the first embodiment shown in Fig. 1, the image-taking device 16 and the moniotor device 18 are employed. However, those devices 16, 18 may be omitted.

In each of the first to fifth embodiments, the tooth-improving apparatus 10, 20, 30, 40, 50 may emit an E wave having a resonance wavelength of at least one substance present in at least one of the tooth S and the tooth-improving (or tooth-coating) material 6, 36. In this case, it is possible to measure, in advance, the resonance wavelength of at least one substance present in the tooth S and the tooth-improving material 6, 36 and adjust the tooth-improving apparatus 10, 20, 30, 40, 50 to emit an E wave having one or more measured resonance wavelengths. The at least one substance present in the tooth S and/or the tooth-improving material 6, 36 may be a substance, other than hydroxyapatite crystal, which constitutes the enamel of the tooth S, such as water, CO₂, or carbonic substance; or a substance, other than hydroxyapatite crystal, which constibutes the dentin of the tooth S, such as water, CO₂, or carbonic substance (in particular, collagen). The tooth S also includes free N-H. The E wave emitted by the apparatus 10, 20, 30, 40, 50 may not have a "peak" or maximum resonance wavelength of a certain substance but may have a "half" or intermediate resonance wavelength. For example, the resonance wavelength of P-O falls in the range of 7.6 to 10.1 µm, preferably in the range of 8.1 to 8.4 µm or 9.6 to 10.1 µm; the resonance wavelength of P-H falls in the range of 4.1 to 4.4 µm; the resonance wavelength of Ca(OH)₂ or Ca(CO₃) falls in the range of 5.5 to 10 µm or 2.6 to 3.3 µm (in particular, 2.85 µm); the resonance wavelength of H₂O falls in the range of 2.9 ± 0.5 µm or 6.1 ± 0.5 µm; the resonance wavelength of CO₂ falls in the range of 4.25 ± 0.5 µm; the resonance wavelength of PO₄ ³⁻, HPO₄ ²⁻, or H₂PO₄⁻ falls in the range of 9.0 to 10 µm; the resonance wavelength of OH⁻ (free) falls in the range of 2.7 to 2.8 µm; and the resonance wavelength of NH (free) falls in the range of 2.8 to 3.0 µm or 3.4 to 4.3 µm. Each of the resonance-wavelength ranges may be shifted by a small value, e.g., ± 0.5, depending upon the differences of individual teeth.

It is to be understood that the present invention may be embodied with other changes, improvements, and modifications that may occur to the person skilled in the art without departing from the scope and spirit of the present invention.

## Claims

1. A tooth-improving apparatus, comprising:
an electromagnetic-wave emitter (10, 20, 30, 40, 50) which emits, toward at least one of (a) a tooth (S) and (b) a tooth-improving material (6, 36) which is applied to a surface of the tooth, at least one electromagnetic wave having at least one resonance wavelength of at least one substance present in said at least one of the tooth and the tooth-improving material.

2. An apparatus according to claim 1, wherein the electromagnetic-wave emitter (10, 20, 30, 40, 50) comprises:
an electromagnetic-wave generator (12, 22, 32, 42, 52) which generates an initial electromagnetic wave; and
a resonance device (14, 22, 34, 44, 52) which provides, from the initial electromagnetic wave, the electromagnetic wave having the resonance wavelength.

3. An apparatus according to claim 2, wherein the electromagnetic-wave generator comprises a globar (32) which emits, as the initial electromagnetic wave, a global electromagnetic wave having a plurality of wavelengths including the resonance wavelength, and wherein the resonance device comprises a filter (34) which transmits, from the global electromagnetic wave, only a wave having the resonance wavelength and does not transmit the other waves having the other wavelengths, thereby providing the electromagnetic wave having the resonance wavelength.

4. An apparatus according to claim 2, wherein the resonance device comprises an interference device (14A, 14B, 44A, 44B) which derives, from the initial electromagnetic wave, two waves having a phase difference therebetween and causes the two waves to interfere with each other, thereby providing the electromagnetic wave having the resonance wavelength.

5. An apparatus according to any of claims 1 to 4, wherein the electromagnetic-wave emitter (20, 50) comprises:
a pulse-wave emitter (24, 54) which emits a plurality of pulse waves as the electromagnetic wave having the resonance wavelength; and
interval-control means (24, 54) for changing an interval between the pulse waves emitted by the pulse-wave emitter.

6. An apparatus according to any of claims 1 to 5, further comprising:
an image taking device (16) which takes an image represented by an electromagnetic wave radiated from said at least one of the tooth and the tooth-improving material; and
a monitor device (18) which displays the image taken by the image taking device.

7. An apparatus according to any of claims 1 to 6, wherein the resonance wavelength falls in a range from 8.8 to 10.4 µm.

8. An apparatus according to any of claims 1 to 7, wherein the resonance wavelength is a resonance wavelength, 9.6 µm, of a phosphoric group of a hydroxyapatite crystal present in said at least one of the tooth and the tooth-improving material.

9. A tooth-improving material (6, 36) for being applied to a surface of a tooth and subjected to at least one electromagnetic wave having at least one resonance wavelength of at least one substance present in at least one of the tooth and the material.

10. A material according to claim 9, wherein the tooth-improving material has a fluidity, and wherein said at least one substance comprises at least one of calcium ion and phosphoric ion.

11. A material according to claim 9 or claim 10, wherein said at least one substance comprises a hydroxyapatite crystal.

12. A material according to any of claims 9 to 11, wherein said at least one substance comprises fluorine.

13. A material according to any of claims 9 to 12, wherein the resonance wavelength falls in a range from 8.8 to 10.4 µm.

14. A material according to any of claims 9 to 13, wherein the resonance wavelength is a resonance wavelength, 9.6 µm, of a phosphoric group of the hydroxyapatite crystal.

15. Use of an electromagnetic-wave emitter (10, 20, 30, 40, 50) for emitting, toward at least one of (a) a tooth (S) and (b) a tooth-improving material (6, 36) which is applied to a surface of the tooth, at least one electromagnetic wave having at least one resonance wavelength of at least one substance present in said at least one of the tooth and the material.

16. Use of a tooth-improving material (6, 36) for being applied to a surface of a tooth and subjected to at least one electromagnetic wave having at least one resonance wavelength of at least one substance present in at least one of the tooth and the material.

17. A tooth-coating method, comprising the steps of:
coating a tooth (S) with a tooth-coating material (6, 36), and
emitting, toward the tooth-coating material, at least one electromagnetic wave having at least one resonance wavelength of at least one substance present in at least one of the tooth and the material.
